# EUROPEAN PATENT SPECIFICATION

(11) **EP 0 868 523 B1**
(45) Date of publication and mention of the grant of the patent: **18.05.2005**
(21) Application number: 96941597.5
(22) Date of filing: 18.12.1996
(51) Int. Cl.: C12N 15/81, C12N 15/62

(54) **VECTOR FOR EXPRESSION OF N-TERMINALLY EXTENDED PROTEINS IN YEAST CELL**
VEKTOR ZUR EXPRESSION VON N-TERMINAL VERLÄNGERTEN PROTEINEN IN HEFEZELLEN
VECTEUR POUR L'EXPRESSION DE PROTEINES A PROLONGEMENT N-TERMINAL DANS LES CELLULES DE LEVURE

(30) Priority: 20.12.1995 DK 144995
(43) Date of publication of application: 07.10.1998
(73) Proprietor: NOVO NORDISK A/S, 2880 Bagsvaerd (DK)
(72) Inventor: KJELDSEN, Thomas, Borglum, DK-2830 Virum (DK); BALSCHMIDT, Per, DK-3060 Espergarde (DK); PETTERSSON, Annette, Frost, DK-3520 Farum (DK)
(74) Representative: Secher, Anne
(86) International application number: PCT/DK1996/000542
(87) International publication number: WO 1997/022706

(56) References cited:
- EP-A- 0 116 201
- EP-A- 0 324 274
- WO-A-86/06406
- WO-A-90/10075
- WO-A-95/02059
- WO-A-95/34666
- WO-A-95/35384

## Description

### FIELD OF INVENTION

The present invention relates to polypeptides expressed and processed in a eucaryotic cell, preferably a yeast cell, a DNA construct comprising a DNA sequence encoding such polypeptides, vectors carrying such DNA fragments and cells including yeast cells transformed with the vectors, as well as a process of producing heterologous proteins in yeast.

### BACKGROUND OF THE INVENTION

Yeast organisms produce a number of proteins synthesized intracellularly, but having a function outside the cell Such extracellular proteins are referred to as secreted proteins. These secreted proteins are expressed initially inside the cell in a precursor or a pre-form containing a presequence ensuring effective direction of the expressed product across the membrane of the endoplasmatic reticulum (ER). The presequence, normally named a signal peptide, is generally cleaved off from the desired product during translocation. Once entered in the secretory pathway, the protein is transported to the Golgi apparatus. From the Golgi the protein can follow different routes that lead to compartments such as the cell vacuole or the cell membrane, or it can be routed out of the cell to be secreted to the external medium (Pfeffer, S.R. and Rothman, J.E. Ann.Rev.Biochem. 56 (1987), 829-852.

Several approaches have been suggested for the expression and secretion in yeast of proteins heterologous to yeast. European Publication No. 0088632A describes a process by which proteins heterologous to yeast are expressed, processed and secreted by transforming a yeast organism with an expression vector harbouring DNA encoding the desired protein and a signal peptide, prepating a culture of the transformed organism, growing the culture and recovering the protein from the culture medium. The signal peptide may be the desired protein's own signal peptide, a heterologous signal peptide or a hybrid of a native and a heterologous signal peptide.

A problem encountered with the use of signal peptides heterologous to yeast might be that the heterologous signal peptide does not ensure efficient translocation and/or cleavage of the precursor polypeptide after the signal peptide.

The Saccharomyces cerevisiae MFα1 (α-factor) is synthesized as a prepro form of 165 amino acids comprising a 19 amino acids long signal- or prepeptide followed by a 64 amino acids long "leader" or propeptide, encompassing three N-linked glycosylation sites followed by (LysArg(Asp/Glu, Ala)₂₋₃α-factor)₄ (Kurjan, J. and Herskowitz, I. Cell 30 (1982), 933-943). The signal-leader part of the preproMFα1 has been widely employed to obtain synthesis and secretion of heterologous proteins in S. cerevisiae.

Use of signal/leader peptides homologous to yeast is known from i.a. US Patent No. 4,546,082, European Publications Nos. 0116201A, 0123294A, 0123544A, 0163529A, 0123289A and European Patent No. 0100561 B.

In EP 0123289A utilization of the S. cerevisiae α-factor precursor is described whereas EP 100561 describes the utilization of the Saccharomyces cerevisiae PH05 signal and PCT Publication No. WO 95/02059 describes the utilization of YAP3 signal peptide for secretion of foreign proteins.

US Patent No. 4,546,082 and European Publications Nos. 0016201A, 0123294A, 0123544A and 0163529A describe processes by which the α-factor signal-leader from Saccharomyces cerevisiae (MFα1 or MFα2) is utilized in the secretion process of expressed heterologous proteins in yeast. By fusing a DNA sequence encoding the S. cerevisiae MFα1 signal/leader sequence at the 5' end of the gene for the desired protein secretion and processing of the desired protein was demonstrated.

EP 206783 discloses a system for the secretion of polypeptides from S. cerevisiae whereby the α-factor leader sequence has been truncated to eliminate the four α-factor peptides present on the native leader sequence so as to leave the leader peptide itself fused to a heterologous polypeptide via the α-factor processing site Lys-Arg-Glu-Ala-Glu-Ala. This construction is indicated to lead to an efficient process for production of smaller peptides (less than 50 amino acids). For the secretion and processing of larger polypeptides, the native α-factor leader sequence has been truncated to leave one or two α-factor peptides between the leader peptide and the polypeptide.

A number of secreted proteins are routed so as to be exposed to a proteolytic processing system which can cleave the peptide bond at the carboxy end the two consecutive basic amino acids. This enzymatic activity is in S. cerevisiae encoded by the KEX 2 gene (Julius, D.A. at al., Cell 37 (1984b), 1075). Processing of the product by the KEX 2 protease is needed for the secretion of active S. cerevisiae mating factor α1 (MFα1 or α-factor) but is not involved in the secretion of active S. cerevisiae mating factor a.

Secretion and correct processing of a polypeptide intended to be secreted is obtained in some cases when culturing a yeast organism which is transformed with a vector constructed as indicated in the references given above. In many cases, however, the level of secretion is very low or there is no secretion, or the proteolytic processing may be incorrect or incomplete. As described in PCT Publication No. WO 90/10075 this is believed to be ascribable, to some extent, to an insufficient exposure of the processing site present between the C-terminal end of the leader peptide and the N-terminal end of the heterologous protein so as to render it inaccessible or, at least, less accessible to proteolytic cleavage.

WO 90/10075 describes a yeast expression system with improved processing of a heterologous polypeptide obtained by providing certain modifications near the processing site at the C-terminal end of the leader peptide and/or the N-terminal end of a heterologous polypeptide fused to the leader peptide. Thus, it is possible to obtain a higher yield of the correctly processed protein than is obtainable with unmodified leader peptide-heterologous polypeptide constructions. It is the object of the present invention to provide a novel N-terminal modification of a heterologous polypeptide fused to the leader peptide which ensures a more efficient expression and/or processing as well as improved removal in vitro of the N-terminal extension of heterologous polypeptides.

### SUMMARY OF THE INVENTION

The present invention describes modifications of the N-terminal end of the heterologous polypeptide designed as extensions which can be cleaved off by in vitro proteolysis during or subsequently to purification of the product from the culture media.

The present invention relates to a DNA construct encoding a polypeptide having the following structure
signal peptide-leader peptide-EEAEPK-heterologous protein.

The conventional one-letter code for amino acids in accordance with rules approved (1974) by the IUPAC-IUB Commission of Biochemical Nomenclature is used throughout this description and in the claims.

In the present context, the term "signal peptide" is understood to mean a presequence which is present as an N-terminal sequence on the precursor form of an extracellular protein expressed in yeast. The function of the signal peptide is to allow the heterologous protein to be secreted to enter the endoplasmatic reticulum. The signal peptide is normally cleaved off in the course of this process. The signal peptide may be heterologous or homologous to the yeast organism producing the protein. A preferred signal peptide in this invention is yeast aspartic protease 3 (YAP3) signal peptide or any functional analogue thereof. YAP 3 has been cloned and characterised by Egel-Mitani, M. et al., YEAST, 6, p. 127-137, 1990.

The expression "leader peptide" is understood to indicate a peptide in the form of a propeptide sequence whose function is to allow the heterologous protein to be secreted to be directed from the endoplasmatic reticulum to the Golgi apparatus and further to a secretory vesicle for secretion into the medium, (i.e. exportation of the expressed protein or polypeptide across the cellular membrane and cell wall, if present, or at least through the cellular membrane into the periplasmic space of a cell having a cell wall). Preferably the leader peptide is the LA19 leader peptide disclosed as SEQ ID No. 5 herein.

The expression "heterologous protein" is intended to indicate a protein or polypeptide which is not produced by the host organism in nature, preferably a polypeptide which is not produced by yeast in nature. However, it would be obvious to a person skilled in the art to produce homologous polypeptides, such as glucagon in a mammalian cell, and said polypeptides are included in the meaning of "heterologous protein".

The sequence EEAEPK forms an extension at the N-terminal of the heterologous polypeptide. This extension not only increases the fermentation yield but is protected against dipeptidyl aminopeptidase (DPAP A) processing, resulting in a homogenous N-terminal of the polypeptide. The extension is constructed in such a way that it is resistant to proteolytic cleavage during fermentation so that the N-terminally extended heterologous protein product can be purified from the culture media for subsequent in vitro maturation, e.g. by trypsin or Achromobacter lyticus protease I. The desired in vitro removal of the N-terminal extension EEAEPK is readily achieved by either trypsin or Achromobacter lyticus protease I presumably due to flexibility of the N-terminal extension peptide resulting in an improved yield of the matured heterologous protein.

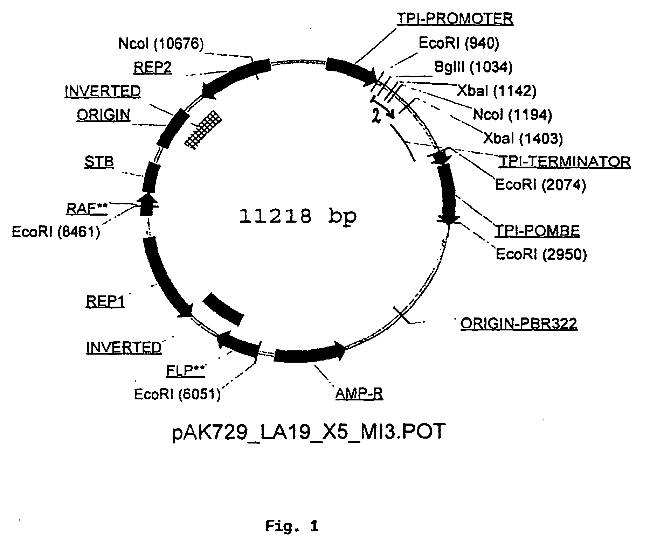

The invention further relates to a recombinant expression vector which is capable of replicating in a eucaryotic cell, preferably a yeast cell, and which carries a DNA construct of the invention. Preferably, the DNA construct comprises a synthetic leader peptide, preferably the LA19 leader peptide. Besides, the invention relates to the DNA construct described in Fig. 1 herein. The invention also relates to a eucaryotic cell, preferably a yeast cell, which is capable of expressing a heterologous protein and which is transformed with a vector of the invention.

Furthermore, the invention relates to a process for producing a heterologous protein in yeast, comprising cultivating the transformed yeast strain in a suitable medium to obtain expression and secretion of the heterologous protein, after which the protein is isolated from the medium.

### BRIEF DESCRIPTION OF THE DRAWINGS

The present invention is further illustrated with reference to the appended drawings, wherein
Fig. 1 shows the expression plasmid pAK729 containing genes expressing the N-terminally extended polypeptides of the invention, and the following symbols are used:
   TPI-PROMOTER: Denotes the TPI gene promoter sequence from S. cerevisiae.
   2: Denotes the region encoding a signal/leader peptide (e.g. from the YAP3 signal peptide and LA19 leader peptide in conjunction with the EEAEPK N-terminally extended MI3 insulin precursor.
   TPI-TERMINATOR: Denotes TPI gene terminator sequence of S. cerevisiae.
   TPI-POMBE: Denotes TPI gene from S. pombe.
   Origin: Denotes a sequence from S. cerevisiae 2 µ plasmid including its origin of DNA replication in S. cerevisiae.
   AMP-R: Sequence from pBR322 /pUC13 including the ampicillin resistance gene and an origin of DNA replication in E. coli.

   SEQ ID NO 1. is the DNA and amino acid sequences in pAK729 encoding the YAP3 signal peptide (amino acid No. 1 through 21), LA19 leader peptide (amino acid No. 22 through 64), N-terminal extension EEAEPK (amino acid No. 65 through 70), MI3 insulin precursor B_{chain}(1-29)-Ala-Ala-Lys-A_{chain}(1-21) (amino acid No. 71 through 123).
   SEQ ID NO 2. is the DNA and amino acid sequences in pAK733 encoding the YAP3 signal petide-LA19 leader EEAEPK-Ml1 insulin precursor complex of Example 2.
   SEQ ID NO 3. is the DNA and amino acid sequences in pAK749 encoding the YAP3 signal petide-LA19 leader EEAEPK-MI5 insulin precursor complex of Example 3.
   SEQ ID NO 4. is the DNA and amino acid sequences in pAK866 encoding the YAP3 signal petide-LA19 leader EEAEPK-X14 insulin precursor complex of Example 4.
   SEQ ID NO 5 is the LA19 leader DNA sequence.

### DETAILED DESCRIPTION OF THE INVENTION

The N-terminally extended heterologous protein produced by the method of the invention may be any protein which may advantageously be produced in a eucaryotic cell, preferably a yeast cell. Examples of such proteins are aprotinin, tissue factor pathway inhibitor or other protease inhibitors, and insulin or insulin precursors, insulin analogues, insulin-like growth factor I or II, human or bovine growth hormone, interleukin, tissue plasminogen activator, transforming growth factor a or b, glucagon, glucagon-like peptide 1 (GLP-1), glucagon-like peptide 2 (GLP-2), GRPP, Factor VII, Factor VIII, Factor XIII, platelet-derived growth factor, enzymes, such as lipases, or a functional analogue of any one of these proteins. In the present context, the term "functional analogue" is meant to indicate a polypeptide with a similar function as the native protein (this is intended to be understood as relating to the nature rather than the level of biological activity of the native protein). The polypeptide may be structurally similar to the native protein and may be derived from the native protein by addition of one or more amino acids to either or both the C- and N-terminal end of the native protein, substitution of one or more amino acids at one or a number of different sited in the native amino acid sequence, deletion of one or more amino acids at either or both ends of the native protein or at one or several sites in the amino acid sequence, or insertion of one or more amino acids at one or more sites in the native amino acid sequence. Such modifications are well known for several of the proteins mentioned above.

By "a precursor of insulin" or "a precursor an insulin analogue" is to be understood a single-chain polypeptide, including proinsulin, which by one or more subsequent chemical and/or enzymatical processes can be converted to a two-chain insulin or insulin analogue molecule having the correct establishment of the three disulphide bridges as found in natural human insulin. Examples of insulins or insulin analogues which can be produced in this way are human insulin, preferably des(B30) human insulin, porcine insulin; and insulin analogues wherein at least one Lys or Arg is present, preferably insulin analogues wherein Phe^{B1} has been deleted, insulin analogues wherein the A-chain and/or the B-chain have an N-terminal extension and insulin analogues wherein the A-chain and/or the B-chain have a C-terminal extension. Other preferred insulin analogues are such wherein one or more of the amino acid residues, preferably one, two, or three of them, have been substituted by another codable amino acid residue. Thus in position A21 a parent insulin may instead of Asn have an amino acid residue selected from the group comprising Ala, Gln, Glu Gly, His, Ile, Leu, Met, Ser, Thr, Trp, Tyr or Val, in particular an amino acid residue selected from the group comprising Gly, Ala, Ser, and Thr. The insulin analogues may also be modified by a combination of the changes outlined above. Likewise, in position B28 a parent insulin may instead of Pro have an amino acid residue selected from the group comprising Asp, Lys etc., and in position B29 a parent insulin may instead of Lys have the amino acid Pro. The expression "a codable amino acid residue" as used herein designates an amino acid residue which can be coded for by the genetic code, i. e. a triplet ("codon") of nucleotides.

Most preferred insulin precursors are MI1, B(1-29)-A(1-21); MI3, B(1-29)-Ala-Ala-Lys-A(1-21) (as described in e.g. EP 163 529); X14, B(1-27-Asp-Lys)-Ala-Ala-Lys-A(1-21) (as described in e.g. PCT publication No. 95/00550); B(1-27-Asp-Lys)-A(1-21); B(1-27-Asp-Lys)-Ser-Asp-Asp-Ala-Lys-A(1-21); B(1-29)-Ala-Ala-Arg-A(1-21) (as described in e.g. PCT Publication No. 95/07931); MI5, B(1-29)-Ser-Asp-Asp-Ala-Lys-A(1-21); and B(1-29)-Ser-Asp-Asp-Ala-Arg-A(1-21).

The DNA construct of the invention encoding the polypeptide of the invention may be prepared synthetically by established standard methods, e.g. the phosphoamidite method described by S.L. Beaucage and M.H. Caruthers, Tetrahedron Letters 22, 1981, pp. 1859-1869, or the method described by Matthes et al., EMBO Journal 3, 1984, pp. 801-805. According to the phosphoamidite method, oligonucleotides are synthesized, e.g. in an automatic DNA synthesizer, purified, duplexed and ligated to form the synthetic DNA construct. A currently preferred way of preparing the DNA construct is by polymerase chain reaction (PCR), e.g. as described in Sambrook et al., Molecular Cloning: A Laboratory Manual, Cold Spring Harbor, NY, 1989).

The DNA construct of the invention may also be of genomic or cDNA origin, for instance obtained by preparing a genomic or cDNA library and screening for DNA sequences coding for all or part of the polypeptide or the invention by hybridization using synthetic oligonucleotide probes in accordance with standard techniques (cf. Sambrook et al., Molecular Cloning: A Laboratory Manual, Cold Spring Harbor, 1989). In this case, a genomic or cDNA sequence encoding a signal and leader peptide may be joined to a genomic or cDNA sequence encoding the heterologous protein, after which the DNA sequence may be modified at a site corresponding to the amino acid sequence EEAEPK of the polypeptide, by inserting synthetic oligonucleotides encoding the desired amino acid sequence for homologous recombination in accordance with well-known procedures or preferably generating the desired sequence by PCR using suitable oligonucleotides.

Finally, the DNA construct may be of mixed synthetic and genomic, mixed synthetic and cDNA or mixed genomic and cDNA origin prepared by annealing fragments of synthetic, genomic or cDNA origin (as appropriate), the fragments corresponding to various parts of the entire DNA construct, in accordance with standard techniques. Thus, it may be envisaged that the DNA sequence encoding the heterologous protein may be of genomic or cDNA origin, while the sequence encoding the signal and leader peptide as well as the sequence encoding the N-terminal extension EEAEPK may be prepared synthetically.

In one aspect, the invention relates to a recombinant expression vector which is capable of replicating in yeast and which carries a DNA construct encoding the above-defined polypeptide. The recombinant expression vector may be any vector which is capable of replicating in yeast organisms. In the vector, the DNA sequence encoding the polypeptide of the invention should be operably connected to a suitable promoter sequence. The promoter may be any DNA sequence which shows transscriptional activity in yeast and may be derived from genes encoding proteins either homologous or heterologous to yeast. The promoter is preferably derived from a gene encoding a protein homologous to yeast. Examples of suitable promoters are the Saccharomyces cerevisiae Mα1, TPI, ADH or PGK promoters.

The DNA sequence encoding the polypeptide of the invention may also be operably connected to a suitable terminator, e.g. the TPI terminator (cf. T. Alber and G. Kawasaki, J. Mol. Appl. Genet. 1, 1982, pp. 419-434).

The recombinant expression vector of the invention comprises a DNA sequence enabling the vector to replicate in yeast. Examples of such sequences are the yeast plasmid 2µ replication genes REP 1-3 and origin of replication. The vector may also comprise a selectable marker, e.g. the Schizosaccharomyces pompe TPI gene as described by P.R. Russell, Gene 40, 1985, pp. 125-130.

The procedures used to ligate the DNA sequences coding for the polypeptide of the invention, the promoter and the terminator, respectively, and to insert them into suitable yeast vectors containing the information necessary for yeast replication, are well known to persons skilled in the art (cf., for instance, Sambrook et al., op.cit.). It will be understood that the vector may be constructed either by first preparing a DNA construct containing the entire DNA sequence coding for the polypeptide of the invention and subsequently inserting this fragment into a suitable expression vector, or by sequentially inserting DNA fragments containing genetic information for the individual elements (such as the signal, leader or heterologous protein) followed by ligation.

The yeast organism or eucaryotic host cell used in the process of the invention may be any suitable yeast organism which, on cultivation, produces large amounts of the heterologous protein or polypeptide in question. Examples of suitable yeast organisms may be strains selected from the yeast species Saccharomyces cerevisiae, Saccharomyces kluyveri, Schizosaccharomyces pombe, Saccharomyces uvarum, Kluyveromyces lactis, Hansenula polymorpha, Pichia pastoris, Pichia methanolica, Pichia kluyveri, Yarrowia lipolytica, Candida sp., Candida utilis, Candida cacaoi, Geotrichum sp., and Geotrichum fermentans, preferably the yeast species Saccharomyces cerevisiae. It is considered obvious for the skilled person in the art to select any other eucaryotic cell, such as cells of the genera Aspergillus, Streptomyces, insect cells or a mammalian cell, as the host organism.

The transformation of the yeast cells may for instance be effected by protoplast formation followed by transformation in a manner known per se. The medium used to cultivate the cells may be any conventional medium suitable for growing yeast organisms. The secreted heterologous protein, a significant proportion of which will be present in the medium in correctly processed form, may be recovered from the medium by conventional precedures including separating the yeast cells from the medium by centrifugation or filtration, precipitating the proteinaceous components of the supernatant or filtrate by means of a salt, e.g. ammonium sulphate, followed by purification by a variety of chromatographic procedures, e.g. ion exchange chromatography, affinity chromatography, or the like. When the protein is secreted to the periplasmic space, the cells are disrupted enzymatically or mechanically.

After secretion to the culture medium or the periplasmic space, the protein may be subjected to various procedures to remove the sequence EEAEPK.

The extension is found to be stably attached to the heterologous protein during fermentation, protecting the N-terminal of the heterologous protein against the proteolytic activity of yeast proteases such as DPAP. The presence of an N-terminal extension on the heterologous protein may also serve as a protection of the N-terminal amino group of the heterologous protein during chemical processing of the protein, i.e. it may serve as a substitute for a BOC or similar protecting group. In such cases the amino acid sequence EEAEPK may be removed from the recovered heterologous protein by means of a proteolytic enzyme which is specific for a basic amino acid (i.e. K (Lys)) so that the terminal extension is cleaved off at the K. Examples of such proteolytic enzymes are trypsin or Achromobacter lyticus protease I.

The present invention is described in further detail in the following examples which are not in any way intended to limit the scope of the invention as claimed.

### Example 1. Construction of the yeast strain yAK729 expressing the EEAEPK-MI3 insulin precursor

A synthetic gene coding for the N-terminal extension of N-terminally extended insulin precursor EEAEPK-MI3 was constructed using the Polymerase Chain Reaction (PCR).

The following 2 oligonucleotides were synthesized:

#672 5' -TCTCCATGGCTAAGAGAGAAGAAGCTGAACCAAAGTTCGTT-3'

#2785 5' -AATTTATTTTACATAACACTAG-3'

Oligonucleotides were synthesized using an automatic DNA synthesizer (applied Biosystems model 380A) using phosphoamidite chemistry and commercially available reagents (Beaucage, S.L. and Caruthers, M.H., Tetrahedron letters 22 (1981) 1859-1869).

The following PCR was performed using the Pwo DNA Polymerase (Boehringer Mannheim GmbH, Sandhoefter Strasse 116, Mannheim, Germany) according to the manufacturer's instructions and The PCR mix was overlayed with 100 µl mineral oil (Sigma Chemical Co., St. Louis MO, USA)

### PCR

5 µl oligonucleotide #672 (50 pmol)
5 µl oligonucleotide #2785 (50 pmol)
10 µl 10X PCR buffer
8 µl dNTP mix
0.5 µl Pwo enzyme
0.5 µl pAK680 plasmid as template (0.2 ug DNA)
71 µl dest. water

A total of 12 cycles were performed, one cycle was 94 C for 45 se.; 40 C for 1 min; 72 C for 1.5 min. The PCR mixture was then loaded onto a 2.5% agarose gel and electroforese was performed using standard techniques (Sambrook J, Fritsch El and Maniatis T, Molecular cloning, Cold Spring harbour laboratory press, 1989). The resulting DNA fragment was cut out of the agarose gel and isolated by the Gene Clean Kit (Bio 101 inc., PO BOX 2284, La Jolla, CA 92038, USA) according to the manufacturer's instructions.

The purified PCR DNA fragment was dissolved in 14 µl of water and restriction endonucleases buffer and cut with the restriction endonucleases Ncol and Xbal according to standard techniques (Sambrook J, Fritsch EF and Maniatis T, Molecular cloning, Cold spring Harbour laboratory press, 1989). The Ncol-Xbal DNA fragment on 209 nucleotide base pairs was subjected to agarose electroforese and purified using The Gene Clean Kit as described.

The plasmid pAK721 (see Fig. 1) was cut with the restriction endonucleases BgIII and Xbal and the vector fragment of 10849 nucleotide base pairs isolated using The Gene Clean Kit as described. The plasmid pAK721 was cut with the restriction endonucleases BgIII and Ncol and the DNA fragment of 160 nucleotide base pairs isolated using The Gene Clean Kit as described. The three DNA fragments was ligated together using T4 DNA ligase and standard conditions (Sambrook J, Fritsch EF and Maniatis T, Molecular cloning, Cold spring Harbour laboratory press, 1989). The ligation mix was then transformed into a competent E. coli strain (R-, M+) followed by selection with ampicillin resistance.

Plasmid from the resulting E. coli was isolated using standard techniques (Sambrook J, Fritsch EF and Maniatis T, Molecular cloning, Cold spring Harbour laboratory press, 1989), and checked for insert with appropriate restriction endonucleases i.e. Nco I and Xbal. The selected plasmid was shown by DNA sequence analyse (Sequenase, U.S. Biochemical Corp., USA) to encode the correct DNA sequence for the EEAEPK-MI3 insulin precursor DNA and to be inserted after the DNA encoding the synthetic LA19 leader DNA. The plasmid was named pAK729.

The DNA sequence encoding the YAP3 signal peptide-LA19 leader EEAEPK-MI3 insulin precursor complex is shown in SEQ ID NO. 1.

The plasmid pAK721 was transformed into S. cerevisiae strain MT663 as described in PCT/DK95/00250 and the resulting strain was named yAK721.

The yeast expression plasmid pAK729 is of the C-POT type (see Fig. 1) and is similar to those described in WO EP 171 142, which contain the Schizosaccharomyces pombe triose phosphate isomerase gene (POT) for plasmid selection and stabilisation in S. cerevisiae. pAK729 also contain the S. cerevisiae triose phosphate isomerase promoter and terminator. The promoter and terminator are similar to those described in the plasmid pKFN1003 (described in WO 90/100075) as are all sequences in plasmid except the sequence between the EcoRIXbaI fragment encoding the YAP3 signal peptide-LA19 leader peptide-EEAEPK-MI3 insulin precursor.

### Example 2. Construction of the yeast strain yAK733 expressing the EEAEPK-MI1 insulin precursor

A synthetic gene coding for the N-terminal extension of N-terminally extended insulin precursor EEAEPK-MI1 was constructed by combining various DNA fragment encoding leader and extension and insulin precursor.

The plasmid pAK721 ( see Fig. 1 ) was cut with the restriction endonucleases BgIII and Xbal and the vector fragment of 10849 nucleotide basepars isolated using The Gene Clean Kit as described.

The plasmid pAK730 was cut with the restriction endonucleases HindIII and Xbal and the DNA fragment of 131 nucleotide basepars isolated using The Gene Clean Kit as described.

The plasmid pAK729 was cut with the restriction endonucleases BgIII and HindIII and the DNA fragment of 229 nucleotide was isolated as described above.

The three DNA fragments was ligated together using T4 DNA ligase and standard conditions (Sambrook J, Fritsch EF and Maniatis T, Molecular cloning, Cold spring Harbour laboratory press, 1989). The ligation mix was then transformed into a competent E. coli strain (R-, M+) followed by selection with ampicillin resistance. Plasmid from the resulting E. coli was isolated using standard techniques (Sambrook J, Fritsch EL and Maniatis T, Molecular cloning, Cold spring Harbour laboratory press, 1989), and checked for insert with appropriate restrictions endonucleases. The selected plasmid was shown by DNA sequence analyse (Sequenase, U.S. Biochemical Corp., USA) to encode the correct DNA sequence for the EEAEPK-MI1 insulin precursor DNA and to be inserted after the DNA encoding the synthetic LA19 leader and extension DNA. The plasmid was named pAK733.

The DNA sequence encoding the YAP3 signal petide-LA19 leader EEAEPK-MI1 insulin precursor complex is shown in SEQ ID NO 2.

The plasmid pAK733 was transformed into S. cerevisiae strain MT663 as described in PCT/DK95/00250 and the resulting strain was named yAK733. The yeast expression plasmid pAK733 is of the C-POT type (see Fig. 1) and is similar to those described in WO EP 171 142, which contains the Schizosaccharomyces pombe triose phosphate isomerase gene (POT) for plasmid selection and stabilisation in S.cerevisiae. pAK733 also contain the S. cerevisiae triose phosphate isomerase promoter and terminator. The promoter and terminator are similar to those described in the plasmid pKFN1003 (described in WO 90/100075) as are all sequences in plasmid except the sequence between the EcoRI-Xbal fragment encoding the YAP3 signal peptide- LA19 leader-EEAEPK-MI1 insulin precursor.

### Example 3. Construction of the yeast strain yAK749 expressing the EEAEPK-MI5 insulin precursor

A synthetic gene coding for the N-terminal extension of N-terminally extended insulin precursor EEAEPK-MI5 was constructed by combining various DNA fragment encoding leader and extension and insulin precursor.

The plasmid pAK743 was cut with the restriction endonucleases BgIII and Nhel and the vector fragment of 10757 nucleotide basepars isolated using The Gene Clean Kit as described.

The plasmid pAK405 was cut with the restriction endonucleases HindIII and Nhel and the DNA fragment of 238 nucleotide basepars isolated using The Gene Clean Kit as described.

The plasmid pAK729 was cut with the restriction endonucleases BgIII and HindIII and the DNA fragment of 229 nucleotide was isolated as described above.

The three DNA fragments was ligated together using T4 DNA ligase and standard conditions (Sambrook J, Fritsch EF and Maniatis T, Molecular cloning, Cold spring Harbour laboratory press, 1989). The ligation mix was then transformed into a competent E. coli strain (R-, M+) followed by selection with ampicillin resistance. Plasmid from the resulting E. coli was isolated using standard techniques (Sambrook J, Fritsch EL and Maniatis T, Molecular cloning, Cold spring Harbour laboratory press, 1989), and checked for insert with appropriate restrictions endonucleases. The selected plasmid was shown by DNA sequence analyse (Sequenase, U.S. Biochemical Corp., USA) to encode the correct DNA sequence for the EEAEPK-MI5 insulin precursor DNA and to be inserted after the DNA encoding the synthetic LA19 leader and extension DNA. The plasmid was named pAK749.

The DNA sequence encoding the YAP3 signal petide-LA19 leader EEAEPK-MI5 insulin precursor complex are shown in SEQ ID NO 3.

The plasmid pAK749 was transformed into S. cerevisiae strain MT663 as described in PCT/DK95/00250 and the resulting strain was named yAK749. The yeast expression plasmid pAK749 is of the C-POT type (see Fig. 1) and is similar to those described in WO EP 171 142, which contains the Schizosaccharomyces pombe triose phosphate isomerase gene (POT) for plasmid selection and stabilisation in S.cerevisiae. pAK749 also contain the S. cerevisiae triose phosphate isomerase promoter and terminator. The promoter and terminator are similar to those described in the plasmid pKFN1003 (described in WO 90/100075) as are all sequences in plasmid except the sequence between the EcoRI-XbaI fragment encoding the YAP3 signal peptide- LA19 leader-EEAEPK-MI5 insulin precursor.

### Example 4. Construction of the yeast strain yAK866 expressing the EEAEPK-X14 insulin precursor

A synthetic gene coding for the N-terminal extension of N-terminally extended insulin precursor EEAEPK-X14 was constructed by combining various DNA fragment encoding leader and extension and X14 insulin precursor.

The plasmid pAK743 was cut with the restriction endonucleases BgIII and Nhel and the vector fragment of 10757 nucleotide basepars isolated using The Gene Clean Kit as described.

The plasmid pAK602 was cut with the restriction endonucleases HindIII and Nhel and the DNA fragment of 232 nucleotide basepars isolated using The Gene Clean Kit as described.

The plasmid pAK729 was cut with the restriction endonucleases BgIII and Hindlll and the DNA fragment of 229 nucleotide was isolated as described above.

The three DNA fragments was ligated together using T4 DNA ligase and standard conditions (Sambrook J, Fritsch EF and Maniatis T, Molecular cloning, Cold spring Harbour laboratory press, 1989). The ligation mix was then transformed into a competent E. coli strain (R-, M+) followed by selection with ampicillin resistance. Plasmid from the resulting E. coli was isolated using standard techniques (Sambrook J, Fritsch EL and Maniatis T, Molecular cloning, Cold spring Harbour laboratory press, 1989), and checked for insert with appropriate restrictions endonucleases. The selected plasmid was shown by DNA sequence analyse (Sequenase, U.S. Biochemical Corp., USA) to encode the correct DNA sequence for the EEAEPK-X14 insulin precursor DNA and to be inserted after the DNA encoding the synthetic LA19 leader and extension DNA. The plasmid was named pAK866.

The DNA sequence encoding the YAP3 signal petide-LA19 leader EEAEPK-X14 insulin precursor complex are shown in SEQ ID NO. 4.

The plasmid pAK866 was transformed into S. cerevisiae strain MT663 as described in PCT/DK95/00250 and the resulting strain was named yAK866. The yeast expression plasmid pAK866 is of the C-POT type (see Fig. 1) and is similar to those described in WO EP 171 142, which contains the Schizosaccharomyces pombe triose phosphate isomerase gene (POT) for plasmid selection and stabilisation in S.cerevisiae. pAK866 also contain the S. cerevisiae triose phosphate isomerase promoter and terminator. The promoter and terminator are similar to those described in the plasmid pKFN1003 (described in WO 90/100075) as are all sequences in plasmid except the sequence between the EcoRI-Xbal fragment encoding the YAP3 signal peptide-LA19 leader-EEAEPK-X14 insulin precursor.
SEQ ID NO 1 
1
M K L K T V R S A V L S
BglII
~~~~~~
ATGAAA CTGAAAACTG TAAGATCTGC GGTCCTTTCG
TACTTT GACTTTTGAC ATTCTAGACG CCAGGAAAGC

S L F A S Q V L G Q P I D D T E S
TCACTCTTTG CATCTCAGGT CCTTGGCCAA CCAATTGACG ACACTGAATC
AGTGAGAAAC GTAGAGTCCA GGAACCGGTT GGTTAACTGC TGTGACTTAG

N T T S V N L M A D D T E S R F
XbaI
~~~~~~
TAACACTACT TCTGTCAACT TGATGGCTGA CGACACTGAA TCTAGATTCG
ATTGTGATGA AGACAGTTGA ACTACCGACT GCTGTGACTT AGATCTAAGC

A T N T T L A L D V V N L I S M A
NcoI
~~~~~~
CTACTAACAC TACTTTGGCT TTGGATGTTG TTAACTTGAT CTCCATGGCT
GATGATTGTG ATGAAACCGA AACCTACAAC AATTGAACTA GAGGTACCGA

K R E E A E P K F V N Q H L C G S
AAGAGAGAAG AAGCTGAACC AAAGTTCGTT AACCAACACT TGTGCGGTTC
TTCTCTCTTC TTCGACTTGG TTTCAAGCAA TTGGTTGTGA ACACGCCAAG

H L V E A L Y L V C G E R G F F
CCACTTGGTT GAAGCTTTGT ACTTGGTTTG CGGTGAAAGA GGTTTCTTCT
GGTGAACCAA CTTCGAAACA TGAACCAAAC GCCACTTTCT CCAAAGAAGA

Y T P K A A K G I V E Q C C T S I
ACACTCCTAA GGCTGCTAAG GGTATTGTCG AACAATGCTG TACCTCCATC
TGTGAGGATT CCGACGATTC CCATAACAGC TTGTTACGAC ATGGAGGTAG
123
C S L Y Q L E N Y C N *
TGCTCCTTGT ACCAATTGGA AAACTACTGC AACTAGACGC AGCCCGCAGG
ACGAGGAACA TGGTTAACCT TTTGATGACG TTGATCTGCG TCGGGCGTCC

XbaI
~~~~~~
CTCTAGA
GAGATCT
SEQ ID NO 2 
EcoRI
~~~~~~~
901 TTCTTGCTTA AATCTATAAC TACAAAAAAC ACATACAGGA ATTCCATTCA
AAGAACGAAT TTAGATATTG ATGTTTTTTG TGTATGTCCT TAAGGTAAGT

951 AGAATAGTTC AAACAAGAAG ATTACAAACT ATCAATTTCA TACACAATAT
TCTTATCAAG TTTGTTCTTC TAATGTTTGA TAGTTAAAGT ATGTGTTATA

+1 M K L K T V R S A V L S
BglII
~~~~~~
1001 AAACGATTAA AAGAATGAAA CTGAAAACTG TAAGATCTGC GGTCCTTTCG
TTTGCTAATT TTCTTACTTT GACTTTTGAC ATTCTAGACG CCAGGAAAGC

+1 S L F A S Q V L G Q P I D D T E S
StyI
~~~~~~
1051 TCACTCTTTG CATCTCAGGT CCTTGGCCAA CCAATTGACG ACACTGAATC
AGTGAGAAAC GTAGAGTCCA GGAACCGGTT GGTTAACTGC TGTGACTTAG

+1 N T T S V N L M A D D T E S R F
XbaI
~~~~~~
1101 TAACACTACT TCTGTCAACT TGATGGCTGA CGACACTGAA TCTAGATTCG
ATTGTGATGA AGACAGTTGA ACTACCGACT GCTGTGACTT AGATCTAAGC

+1 A T N T T L A L D V V N L I S M A
StyI
~~~~~~
NcoI
~~~~~~
1151 CTACTAACAC TACTTTGGCT TTGGATGTTG TTAACTTGAT CTCCATGGCT
GATGATTGTG ATGAAACCGA AACCTACAAC AATTGAACTA GAGGTACCGA

+1 K R E E A E P K F V N Q H L C G S
1201 AAGAGAGAAG AAGCTGAACC AAAGTTCGTT AACCAACACT TGTGCGGTTC
TTCTCTCTTC TTCGACTTGG TTTCAAGCAA TTGGTTGTGA ACACGCCAAG

+1 H L V E A L Y L V C G E R G F F
HindIII
~~~~~~
1251 CCACTTGGTT GAAGCTTTGT ACTTGGTTTG CGGTGAAAGA GGTTTCTTCT
GGTGAACCAA CTTCGAAACA TGAACCAAAC GCCACTTTCT CCAAAGAAGA

+1 Y T P K G I V E Q C C T S I C S L
Bsu36I
~~~~~~~~~
1301 ACACTCCTAA GGGTATTGTC GAACAATGCT GTACCTCCAT CTGCTCCTTG
TGTGAGGATT CCCATAACAG CTTGTTACGA CATGGAGGTA GACGAGGAAC

+1 Y Q L E N Y C N *
XbaI
~~~~~~
1351 TACCAATTGG AAAACTACTG CAACTAGACG CAGCCCGCAG GCTCTAGAAA
ATGGTTAACC TTTTGATGAC GTTGATCTGC GTCGGGCGTC CGAGATCTTT
SEQ ID NO 3. 
EcoRI
~~~~~~~
901 TTCTTGCTTA AATCTATAAC TACAAAAAAC ACATACAGGA ATTCCATTCA
AAGAACGAAT TTAGATATTG ATGTTTTTTG TGTATGTCCT TAAGGTAAGT

951 AGAATAGTTC AAACAAGAAG ATTACAAACT ATCAATTTCA TACACAATAT
TCTTATCAAG TTTGTTCTTC TAATGTTTGA TAGTTAAAGT ATGTGTTATA

+1 M K L K T V R S A V L S
BglII
~~~~~~
1001 AAACGATTAA AAGAATGAAA CTGAAAACTG TAAGATCTGC GGTCCTTTCG
TTTGCTAATT TTCTTACTTT GACTTTTGAC ATTCTAGACG CCAGGAAAGC

+1 S L F A S Q V L G Q P I D D T E S
StyI
~~~~~~
1051 TCACTCTTTG CATCTCAGGT CCTTGGCCAA CCAATTGACG ACACTGAATC
AGTGAGAAAC GTAGAGTCCA GGAACCGGTT GGTTAACTGC TGTGACTTAG

+1 N T T S V N L M A D D T E S R F
XbaI
~~~~~~
1101 TAACACTACT TCTGTCAACT TGATGGCTGA CGACACTGAA TCTAGATTCG
ATTGTGATGA AGACAGTTGA ACTACCGACT GCTGTGACTT AGATCTAAGC

+1 A T N T T L A L D V V N L I S M A
StyI
~~~~~~
NcoI
~~~~~~
1151 CTACTAACAC TACTTTGGCT TTGGATGTTG TTAACTTGAT CTCCATGGCT
GATGATTGTG ATGAAACCGA AACCTACAAC AATTGAACTA GAGGTACCGA

+1 K R E E A E P K F V N Q H L C G S
1201 AAGAGAGAAG AAGCTGAACC AAAGTTCGTT AACCAACACT TGTGCGGTTC
TTCTCTCTTC TTCGACTTGG TTTCAAGCAA TTGGTTGTGA ACACGCCAAG

+1 H L V E A L Y L V C G E R G F F
HindIII
~~~~~~
1251 CCACTTGGTT GAAGCTTTGT ACTTGGTTTG CGGTGAAAGA GGTTTCTTCT
GGTGAACCAA CTTCGAAACA TGAACCAAAC GCCACTTTCT CCAAAGAAGA

+1 Y T P K S D D A K G I V E Q C C T
1301 ACACTCCTAA GTCTGACGAT GCTAAGG[?]TA TTGTCGAGCA ATGCTGTACC
TGTGAGGATT CAGACTGCTA CGATTCC[?]AT AACAGCTCGT TACGACATGG

+1 S I C S L Y Q L E N Y C N *
1351 TCCATCTGCT CCTTGTACCA ATTGGAAAAC TACTGCAACT AGACGCAGCC
AGGTAGACGA GGAACATGGT TAACCTTTTG ATGACGTTGA TCTGCGTCGG

XbaI
~~~~~~~
1401 CGCAGGCTCT AGAAACTAAG ATTAATATAA TTATATAAAA ATATTATCTT
GCGTCCGAGA TCTTTGATTC TAATTATATT AATATATTTT TATAATAGAA
SEQ ID NO 4 
EcoRI
~~~~~~~
901 TTCTTGCTTA AATCTATAAC TACAAAAAAC ACATACAGGA ATTCCATTCA
AAGAACGAAT TTAGATATTG ATGTTTTTTG TGTATGTCCT TAAGGTAAGT

951 AGAATAGTTC AAACAAGAAG ATTACAAACT ATCAATTTCA TACACAATAT
TCTTATCAAG TTTGTTCTTC TAATGTTTGA TAGTTAAAGT ATGTGTTATA

+1 M K L K T V R S A V L S
BglII
~~~~~~
1001 AAACGATTAA AAGAATGAAA CTGAAAACTG TAAGATCTGC GGTCCTTTCG
TTTGCTAATT TTCTTACTTT GACTTTTGAC ATTCTAGACG CCAGGAAAGC

+1 S L F A S Q V L G Q P I D D T E S
StyI
~~~~~~
1051 TCACTCTTTG CATCTCAGGT CCTTGGCCAA CCAATTGACG ACACTGAATC
AGTGAGAAAC GTAGAGTCCA GGAACCGGTT GGTTAACTGC TGTGACTTAG

+1 N T T S V N L M A D D T E S R F
XbaI
~~~~~~
1101 TAACACTACT TCTGTCAACT TGATGGCTGA CGACACTGAA TCTAGATTCG
ATTGTGATGA AGACAGTTGA ACTACCGACT GCTGTGACTT AGATCTAAGC

+1 A T N T T L A L D V V N L I S M A
StyI
NcoI
~~~~~~
1151 CTACTAACAC TACTTTGGCT TTGGATGTTG TTAACTTGAT CTCCATGGCT
GATGATTGTG ATGAAACCGA AACCTACAAC AATTGAACTA GAGGTACCGA

+1 K R E E A E P K F V N Q H L C G S
1201 AAGAGAGAAG AAGCTGAACC AAAGTTCGTT AACCAACACT TGTGCGGTTC
TTCTCTCTTC TTCGACTTGG TTTCAAGCAA TTGGTTGTGA ACACGCCAAG

+1 H L V E A L Y L V C G E R G F F
HindIII
~~~~~~
1251 CCACTTGGTT GAAGCTTTGT ACTTGGTTTG TGGTGAAAGA GGTTTCTTCT
GGTGAACCAA CTTCGAAACA TGAACCAAAC ACCACTTTCT CCAAAGAAGA

+1 Y T D K A A K G I V E Q C C T S I
1301 ACACTGACAA GGCTGCTAAG GGTATTGTTG AACAATGTTG TACCTCTATC
TGTGACTGTT CCGACGATTC CCATAACAAC TTGTTACAAC ATGGAGATAG

+1 C S L Y Q L E N Y C N *
1351 TGTTCTTTGT ACCAATTGGA AAACTACTGT AACTAGACGC AGCCCGCAGG
ACAAGAAACA TGGTTAACCT TTTGATGACA TTGATCTGCG TCGGGCGTCC

XbaI
~~~~~~
1401 CTCTAGACTA AGATTAATAT AATTATATAA AAATATTATC TTCTTTTCTT
GAGATCTGAT TCTAATTATA TTAATATATT TTTATAATAG AAGAAAAGAA
 
XbaI
      ~~~~~~
1401  AGGCTCTAGA AACTAAGATT AATATAATTA TATAAAAATA TTATCTTCTT
      TCCGAGATCT TTGATTCTAA TTATATTAAT ATATTTTTAT AATAGAAGAA
SEQ ID NO 5, LA19 leader
   INFORMATION FOR SEQ ID NO:5:
      (i) SEQUENCE CHARACTERISTICS:
         (A) LENGTH: 43 amino acids
         (B) TYPE: amino acid
         (D) TOPOLOGY: linear
      (ii) MOLECULE TYPE: peptide
      (xi) SEQUENCE DESCRIPTION: SEQ ID NO:67:

Gln Pro Ile Asp Asp Thr Glu Ser Asn Thr Thr Ser Val Asn Leu Met
1               5                   10                  15

Ala Asp Asp Thr Glu Ser Arg Phe Ala Thr Asn Thr Thr Leu Ala Leu
            20                  25                  30

Asp Val Val Asn Leu Ile Ser Met Ala Lys Arg
        35                  40

## Claims

1. A DNA construct encoding a polypeptide having the following structure signal peptide-leader peptide-EEAEPK-heterologous protein.

2. A DNA construct according to the preceding claim, wherein the signal peptide is the yeast aspartic protease 3 (YAP3) signal peptide or a functional analogue thereof.

3. A DNA construct according to any one of the preceding claims, wherein the leader peptide is a synthetic leader peptide, preferably the LA19 leader peptide of SEQ ID NO 5 herein.

4. A DNA construct according to any one of the preceding claims, wherein the heterologous protein is selected from the group consisting of aprotinin, tissue factor pathway inhibitor or other protease inhibitors, and insulin or insulin precursors, insulin analogues, insulin-like growth factor I or II, human or bovine growth hormone, interleukin, tissue plasminogen activator, transforming growth factor a or b, glucagon, glucagon-like peptide 1 (GLP-1), glucagon-like peptide 2 (GLP-2), GRPP, Factor VII, Factor VIII, Factor XIII, platelet-derived growth factor, enzymes, such as lipases, or a functional analogue of any one of these proteins.

5. A DNA construct according to claim 4, wherein the heterologous protein is insulin or an insulin precursor, preferably selected from the group consisting of MI1, B(1-29)-A(1-21); MI3, B(1-29)-Ala-Ala-Lys-A(1-21); X14, B(1-27-Asp-Lys)-Ala-Ala-Lys-A(1-21); B(1-29)-Ala-Ala-Arg-A(1-21); MI5, B(1-29)-Ser-Asp-Asp-Ala-Lys-A(1-21); and B(1-29)-Ser-Asp-Asp-Ala-Arg-A(1-21).

6. A DNA construct according to claim 4, wherein the heterologous protein is insulin or an insulin precursor, preferably the insulin precursor MI3, or a functional analogue thereof.

7. The DNA construct described in SEQ ID NO. 1, 2, 3, or 4 herein.

8. A recombinant expression vector which is capable of replicating in a eukaryotic cell and which carries a DNA construct according to any one of the preceding claims.

9. A recombinant expression vector which is capable of replicating in yeast and which carries a DNA construct according to any one of claims 1 through 7.

10. A yeast cell which is capable of expressing a heterologous protein and which is transformed with a vector according to claim 9.

11. A yeast cell according to claim 10 selected from the species Saccharomyces cerevisiae, Saccharomyces kluyveri, Schizosaccharomyces pombe, Saccharomyces uvarum, Kluyveromyces lactis, Hansenula polymorpha, Pichia pastoris, Pichia methanolica, Pichia kluyveri, Yarrowia lipolytica, Candida sp., Candida utilis, Candida cacaoi, Geotrichum sp., and Geotrichum fermentans, preferably Saccharomyces cerevisiae.

12. A process for producing a heterologous protein, comprising cultivation a yeast cell according to claim 10 or 11 in a suitable medium to obtain expression and secretion of the heterologous protein, after which the protein is isolated from the culture medium.

13. A process according to claim 12, wherein the amino acid sequence EEAEPK is removed from the recovered heterologous protein by a process involving treatment with a proteolytic enzyme which is specific for a basic amino acid.

14. A process according to claim 13, wherein the proteolytic enzyme is selected from the group consisting of trypsin and Achromobacter lyticus protease I.

## Patentansprüche

1. DNA-Konstrukt, das ein Polypeptid mit der folgenden Struktur kodiert: Signalpeptid-Leaderpeptid-EEAEPK-heterologes Protein.

2. DNA-Konstrukt nach dem vorangehenden Anspruch, wobei das Signalpeptid das Signalpeptid Hefeaspartamprotease 3 (YAP3) oder ein funktionelles Analogon davon ist.

3. DNA-Konstrukt nach einem der vorangehenden Ansprüche, wobei das Leaderpeptid ein synthetisches Leaderpeptid, vorzugsweise das Leaderpeptid LA19 der SEQ ID NO. 5 hierin ist.

4. DNA-Konstrukt nach einem der vorangehenden Ansprüche, wobei das heterologe Protein ausgewählt ist aus der Gruppe, bestehend aus Aprotinin, Hemmstoff für den Gewebewachstumsweg oder anderen Proteasehemmstoffen und Insulin oder Insulinvorstufen, Insulinanaloga, insulinartigem Wachstumsfaktor I oder II, menschlichem oder Rinderwachstumshormon, Interleukin, Gewebeplasminogenaktivator, transformierendem Wachstumsfaktor a oder b, Glucagon, glucagonartigem Peptid 1 (GLP-1), glucagonartigem Peptid 2 (GLP-2), GRPP, Faktor VII, Faktor VII, Faktor XIII, Plättchen-abgeleitetem Wachstumsfaktor, Enzymen wie Lipasen oder einem funktionellen Analogon von einem dieser Proteine.

5. DNA-Konstrukt nach Anspruch 4, wobei das heterologe Protein Insulin oder eine Insulinvorstufe, vorzugsweise ausgewählt aus der Gruppe, bestehend aus M11, B(1-29)-A(1-21); M13, B(1-29)-Ala-Ala-Lys-A(1-21); X14, B(1-27-Asp-Lys)-Ala-Ala-Lys-A(1-21); B(1-29)-Ala-Ala-Arg-A(1-21); M15, B(1-29)-Ser-Asp-Asp-Ala-Lys-A(1-21); und B(1-29)-Ser-Asp-Asp-Ala-Arg-A(1-21) ist.

6. DNA-Konstrukt nach Anspruch 4. wobei das heterologe Protein Insulin oder eine Insulinvorstufe, vorzugsweise die Insulinvorstufe M13 oder ein funktionelles Analogon davon ist.

7. DNA-Konstrukt wie in SEQ ID NO. 1, 2, 3 oder 4 hierin beschrieben.

8. Rekombinanter Expressionsvektor, der in einer eukaryotischen Zelle replizieren kann und ein DNA-Konstrukt nach einem der vorangehenden Ansprüche trägt.

9. Rekombinanter Expressionsvektor, der in Hefe replizieren kann und ein DNA-Konstrukt nach einem der Ansprüche 1 bis 7 trägt.

10. Hefezelle, die ein heterologes Protein exprimieren kann und mit einem Vektor nach Anspruch 9 transformiert ist.

11. Hefezelle nach Anspruch 10, ausgewählt aus der Spezies *Saccharomyces cerevisiae, Saccharomyces kluyveri, Schizosaccharomyces pombe, Saccharomyces uvarum, Kluyveromyces lactis, Hansenula polymorpha, Pichia pastoris, Pichia methanolica, Pichia kluyveri, Yarrowia lipolytica*, *Candida sp., Candida utilis, Candida cacaoi, Geotrichum sp*. und *Geotrichum fermentans,* vorzugsweise *Saccharomyces cerevisiae*.

12. Verfahren zur Herstellung eines heterologen Proteins, umfassend das Züchten einer Hefezelle nach Anspruch 10 oder 11 in einem geeigneten Medium zum Erhalt von Expression und Sekretion des heterologen Proteins, wonach das Protein aus dem Kulturmedium isoliert wird.

13. Verfahren nach Anspruch 12, wobei die Aminosäuresequenz EEAEPK von dem gewonnenen heterologen Protein durch ein Verfahren entfernt wird, das die Behandlung mit einem proteolytischen Enzym, das für eine basische Aminosäure spezifisch ist, beinhaltet.

14. Verfahren nach Anspruch 13, wobei das proteolytische Enzym ausgewählt ist aus der Gruppe, bestehend aus Trypsin und der Protease I von *Achromobacter lyticus*.

## Revendications

1. Produit de synthèse d'ADN codant pour un polypeptide ayant la structure suivante peptide signal-peptide leader-EEAEPK-protéine hétérologue.

2. Produit de synthèse d'ADN selon la revendication 1, dans lequel le peptide signal est le peptide signal de la protéase 3 aspartique de levure (YAP3) ou un analogue fonctionnel de celui-ci.

3. Produit de synthèse d'ADN selon l'une quelconque des revendications précédentes, dans lequel le peptide leader est un peptide leader synthétique, de préférence le peptide leader LA19 de la SEQ ID N° : 5.

4. Produit de synthèse d'ADN selon l'une quelconque des revendications précédentes, dans lequel la protéine hétérologue est sélectionnée parmi le groupe constitué d'aprotinine, inhibiteur de la voie du facteur tissulaire ou autres inhibiteurs de protéase, et insuline ou précurseurs d'insuline, analogues d'insuline, facteur de croissance I ou II analogue à l'insuline, hormone de croissance humaine ou bovine, interleukine, activateur tissulaire du plasminogène, facteur de croissance transformant a ou b, glucagon, peptide 1 analogue au glucagon (GLP-1), peptide 2 analogue au glucagon (GLP-2), GRPP, Facteur VII, Facteur VIII, Facteur XIII, facteur de croissance dérivé de plaquettes, enzymes, telles que des lipases, ou un analogue fonctionnel de l'une quelconque de ces protéines.

5. Produit de synthèse d'ADN selon la revendication 4, dans lequel la protéine hétérologue est une insuline ou un précurseur d'insuline, de préférence sélectionnée parmi le groupe constitué de MIl, B(1-29)-A(1-21), MI3, B(1-29)-Ala-Ala-Lys-A(1-21), X14, B(1-27-Asp-Lys)-Ala-Ala-Lys-A(1-21), B(1-29)-Ala-Ala-Arg-A(1-21), MI5, B(1-29)-Ser-Asp-Asp-Ala-Lys-A(1-21), et B(1-29)-Ser-Asp-Asp-Ala-Arg-A(1-21).

6. Produit de synthèse d'ADN selon la revendication 4, dans lequel la protéine hétérologue est une insuline ou un précurseur d'insuline, de préférence le précurseur d'insuline MI3, ou un analogue fonctionnel de celui-ci.

7. Produit de synthèse d'ADN décrit dans la SEQ ID N° : 1, 2, 3 ou 4.

8. Vecteur d'expression recombinant qui est capable de se répliquer dans une cellule eucaryote et qui transporte un produit de synthèse d'ADN selon l'une quelconque des revendications précédentes.

9. Vecteur d'expression recombinant qui est capable de se répliquer dans une levure et qui transporte un produit de synthèse d'ADN selon l'une quelconque des revendications 1 à 7.

10. Cellule de levure qui est capable d'exprimer une protéine hétérologue et qui est transformée à l'aide d'un vecteur selon la revendication 9.

11. Cellule de levure selon la revendication 10, sélectionnée parmi les espèces Saccharomyces cerevisiae, Saccharomyces kluyveri, Schizosaccharomyces pombe, Saccharomyces uvarum, Kluyveromyces lactis, Hansenula polymorpha, Pichia pastoris, Pichia methanolica, Pichia kluyveri, Yarrowia lipolytica, Candida sp., Candida utilis, Candida cacaoi, Geotrichum sp., et Geotrichum fermentans, de préférence Saccharomyces cerevisiae.

12. Procédé pour produire une protéine hétérologue, comportant la culture d'une cellule de levure selon la revendication 10 ou 11, dans un milieu adapté pour obtenir l'expression et la sécrétion de la protéine hétérologue, après quoi la protéine est isolée à partir du milieu de culture.

13. Procédé selon la revendication 12, dans lequel la séquence d'acides aminés EEAEPK est retirée de la protéine hétérologue récupérée par un procédé impliquant le traitement à l'aide d'une enzyme protéolytique qui est spécifique à un acide aminé basique.

14. Procédé selon la revendication 13, dans lequel l'enzyme protéolytique est sélectionnée parmi le groupe constitué de trypsine et de la protéase I de Achromobacter lyticus.
